# EUROPEAN PATENT APPLICATION

(11) **EP 0 695 733 A1**
(43) Date of publication of application: **07.02.1996**
(21) Application number: 95114705.7
(22) Date of filing: 04.07.1991
(51) Int. Cl.: C07C 39/23, C07C 321/26, C07C 37/14, C08F 232/04, C07C 39/17

(54) **Olefin polymers containing bound antioxidant**

(30) Priority: 10.07.1990 US 550373; 02.11.1990 US 608292
(62) Divisional of application: 91201719.1
(71) Applicant: DSM N.V., NL-6411 TE Heerlen (NL)
(72) Inventor: Olivier, Errol Joseph, Baton Rouge, La. 70810 (US); Young, Harold William Jr., Baton Rouge, La. 70810 (US)

(57) **Abstract**

This invention is addressed to olefin polymers having antioxidant properties which are prepared by Ziegler polymerization of one or more monomers containing ethylenic unsaturation along with a polymerizable antioxidant monomer in the form of a substituted norbornene in which one substituent contains a group imparting to the compound antioxidant properties.

## Description

### BACKGROUND OF THE INVENTION

This invention relates to olefin polymers possessing antioxidant properties prepared by Ziegler polymerization of a polymerizable antioxidant monomer with alpha-olefins. This invention further provides a method of introducing functionality into polyolefins by direct Ziegler catalysis without excessive use of co-catalyst or blocking agents.

The development of the now well known Ziegler catalyst system has made it possible to polymerize alpha- olefins to highly useful polymers and copolymers. One of the principal drawbacks to the Ziegler catalyst system is that it cannot be used with monomers containing functional groups which are polar in nature. As a general rule, such polar functional groups have a tendency to react irreversibly with components of the Ziegler catalyst system, thereby lowering the true concentration of the Ziegler catalyst components.

Equally well known is the fact that virtually all polyolefins require stabilization against uncontrolled oxidation which has a tendency to cause undesirable changes in the polymer, including chain scission, cross-linking and discoloration, and thus, adversely change the mechanical and physical properties of the polymer. Extensive search has been undertaken in the area of stabilization, and that research has resulted in the development of a number of antioxidants which impart greater stability to olefin polymers, including elastomeric olefin polymers. A major class of antioxidants which has been developed over the years is the class of hindered phenols.

While molecular antioxidants such as the hindered phenols have achieved wide spread use in the stabilization of a wide variety of polyolefins, they have a tendency under certain conditions of use to migrate out of the polymer which results in the depletion of the antioxidant in the polymer, and consequently the polymer has a tendency to be degraded through oxidation. The use of bound antioxidants which remain in the polymer under conditions which promote the migration of molecular antioxidants has been advocated by (1) Kline, R.H. and Miller, J.P., "Preparation and Activity of Polymerizable Antioxidants for Emulsion Rubbers," Rubber Chemistry and Technology, 46, 96 (1973); (2) Meyer, G.E., Kavchok, R.W. and Naples, F.J., "Emulsion Rubbers Copolymerized with Monomeric Antioxidants," Rubber Chemistry and Technology, 46, 106 (1973); (3) Horvath, J.W., "Bound Antioxidant Stabilized NBR in Automotive Applications," Elastomerics, August, 1979, page 19; (4) Kuczkowski, J.A. and Gillick, J.G., "Polymer-Bound Antioxidants," Rubber Chemistry and Technology, 57, 621 (1984); (5) Engels, H.W. et al., "Effectiveness of New Alkyl-Aryl-p-Phenylenediamines Which Can Be Chemically Bound to Polymers - Model Study," Rubber Chemistry and Technology, 62, 609 (1989); (6) Parker, D.K. and Schulz, G.O., "N-(4-Anilinophenyl)-Methacrylamide, A Polymerizable Amine Antioxidant: Synthesis, Copolymerization, Copolymer Properties, and Performance," Rubber Chemistry and Technology, 62, 732 (1989); (7) Gandek, T.P., Hatton, T.A. and Reid, R.C., "Batch Extraction with Reaction: Phenolic Antioxidant Migration from Polyolefins to Water. 2. Experimental Results and Discussion," Ind. Eng. Chem. Res., 28, 1036 (1989); and (8) Miller, D.E. et al., "Persistent Antioxidants for Polymers Contacting Extractive Media," Rubber World, August 1989, page 13. Such antioxidants are characterized as polymer-bound by reason of the fact that they are chemically attached to the polymer either by way of a grafting reaction or by copolymerization with the olefinic monomers during the production of the polymer itself.

Polymer-bound antioxidants which result from copolymerization with the other monomers have been generally limited to free radical polymerizations, and particularly the free radical emulsion copolymerization of butadiene and acrylonitrile in the production of NBR rubbers. Typical polymer-bound antioxidant monomers include amide or ester derivatives of acrylic or methacrylic acid which can be copolymerized by way of a free radical mechanism with the butadiene and acrylonitrile. While such polymer-bound antioxidants are well suited as monomers in free radical polymerization techniques, they are unsuitable for use in polymerizations catalyzed by the Ziegler catalyst system because their polar groups tend to act as catalyst poisons.

It has been proposed in United States Patent Nos. 3,748,316; 3,796,687 and 4,017,669 to incorporate by copolymerization polar monomers using a Ziegler catalyst system. Specifically, those prior patents suggest certain norbornene compounds having a phenolic group chemically bound thereto as monomers for copolymerization with ethylene and propylene by way of a Ziegler catalyst system. The general teachings of those references include a compound said to have the following general structure: 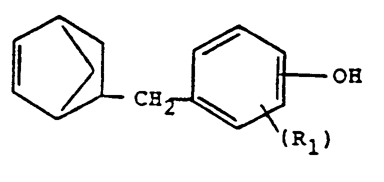
where R₁ is either R or OR and R can be alkyl, aryl or cycloalkyl containing 1-18 carbon atoms or hydrogen.

The teachings of the three patents do not describe any technique by which compounds of that type can be prepared, nor do they describe a polymerization with such a monomer. Therefore, the three patents fail to place those compounds and copolymers prepared therefrom in the
possession of the public. Additionally, it is also important to note that these patents overcome the poisoning effect of polar groups by using an equal molar quantity of aluminum alkyl to polar monomer (not the monomer referred to herein), a very expensive solution, as well as impractical in view of environmental and purity standards of today's rubber and plastics industry. Furthermore, no recognition is given to the potential for the phenolic type polar monomer to impart antioxidant properties to the polar copolymer obtained therefrom.

U.S. Patent No. 4,301,306 describes norbornenyl monophenolic compounds of the general structure below: 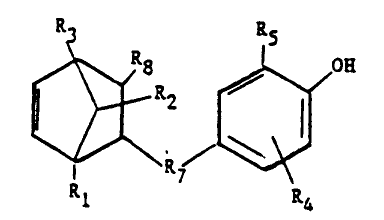
as having a reactive double bond and thereby being polymerizable. This patent teaches neither how to use the reactive norbornenyl phenolic compound in polymerization reactions, nor does it demonstrate that once polymerized, the copolymer thus obtained possesses antioxidant properties.

Norbornenyl monophenolic compounds described above were the subject of U.S. Patent No. 4,355,148 where a ring opening polymerization using a metathesis catalyst produced a polymeric antioxidant composition incorporating the norbornenyl phenolic compound with dicyclopentadiene, norbornene or substituted norbornenes, and optionally an olefin such as 1-hexene.

It is accordingly an object of the present invention to provide polyolefins possessing antioxidant properties prepared by conventional Ziegler polymerization processes whereby an olefin-containing antioxidant is copolymerized with the alpha-olefin or mixture of olefins, and is directly incorporated into the polymer backbone. A further object of the invention is to introduce functionality into the polyolefins by direct Ziegler catalysis without use of special techniques such as blocking groups and excessive use of organo-aluminum co-catalyst.

The concepts of the invention reside in the use of Ziegler-polymerizable antioxidant compounds which are in the form of a substituted norbornene in which the substituent contains an arylgroup imparting to the compound antioxidant properties. Preferred among the antioxidant-imparting substituent are hindered hydroxy-substituted aromatic groups, the most preferred being hindered alkyl-substituted phenols.

The Ziegler-polymerizable antioxidants of the present invention are compounds having the general formula: 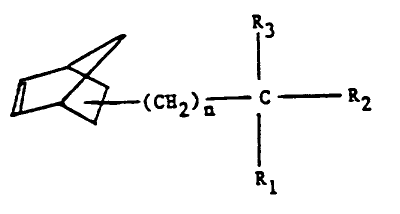
wherein n= 0-3,
R₁ is an anti-oxidant imparting substituent having the formula: 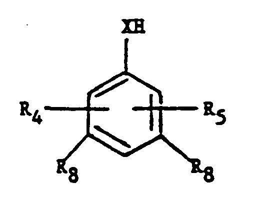
wherein X is selected from a divalent oxygen atom and a divalent sulfur atom; R₄ and R₅ are each independently alkyl containing 1-8 carbon atoms, such as methyl, ethyl, propyl, isopropyl, butyl, t-butyl, pentyl, isopentyl, etc. or additionally R₄ can be hydrogen, R₈ is either hydrogen, methyl, ethyl, or mixtures thereof, R₂ is a hydrogen atom, and R₃, in the above structure, is either hydrogen or an alkylgroup, such as methyl, ethyl, propyl, etc.

A suitable route for synthesis of the monophenolic olefin containing antioxidants of the present invention is disclosed in Layer U.S. Patent No. 4,301,306 and is incorporated herein by reference. Layer uses the well known Diels Alder reaction to form a 2-norbornene substituted in the 5-position with an alkylene or alkenylene phenolic group by reacting a cyclopentadiene with a 4-alkenyl phenol as shown below: 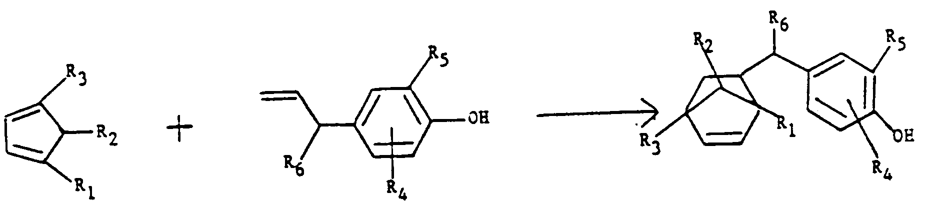
Specific preferred examples would include the following structures: 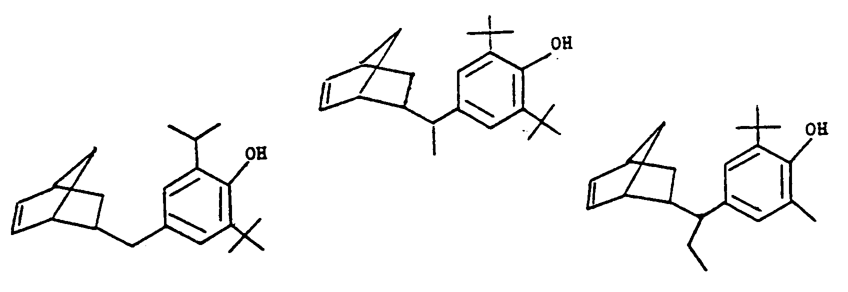
As will be appreciated by those skilled in the art, if use is made of a mixture of phenols, a mixture of thiophenols or a mixture of phenol with a thiophenol, then the product will likely be a mixture of compounds in which the norbornene molecule is substituted with mixed phenols and thiophenols.

The Ziegler polymerizable antioxidants of the present invention find particular utility as co-monomers in the Ziegler catalyzed polymerization of olefins, generally. Thus, the polymerizable antioxidant of the present invention can be copolymerized with any alpha-olefin such as ethylene, propylene, butene or hexene with conjugated or non-conjugated dienes such as butadiene, isoprenes and 1,4-hexadiene and with styrene. In addition, the anti-oxidants of the present invention can be used as co-monomers in the polymerization of olefin mixtures to produce terpolymers, tetrapolymer, etc. The resultant antioxidant- containing polymers can be crystalline or amorphous and find utility broadly, in applications from plastics to elastomerics.

Typical Ziegler catalysts useful in the practice of this invention are comprised of a transition metal compound in which the metal is selected from Group IV-VI of the Periodic Table (as published in CRC Handbook of Chemistry and Physics, by The Chemical Rubber Company, Cleveland, Ohio) used in combination with a base metal alkyl or hydride with at least one carbon to metal bonded group and the metal selected from Group I-III of the Periodic Table. Examples of commonly employed transition metals would include vanadium, titanium, zirconium, cobalt, chromium and nickel. Examples of base metal alkyls commonly used are various organo aluminum compounds, organo aluminum halides, organo zinc compounds or organo magnesium compounds. The transition metal component may be homogeneous or heterogeneous and can be complexed with or used in combination with an electron donating chemicals such as organic esters, amines, phosphines, silicates or others. Alternately, the transition metal can be supported on an inert carrier such as MgO, MgCl₂, silica or alumina. Also suitable in the practice of this invention, particularly for preparation of antioxidant bound polyethylenes, is CrO₃ supported on activated silica-alumina, also known as the "Phillips catalyst."

The Ziegler polymerization reaction with the olefin containing antioxidant monomer is not process limited and can be carried out in solution, slurry, gas phase or any combination of such processes. The choice of process, as known to those skilled in the art, will depend on the type of polyolefin and the specific Ziegler catalyst system.

The active Ziegler catalyst is not adversely effected by the polymerizable antioxidant monomer. Standard Ziegler polymerization techniques, including those for molecular weight control known to those skilled in the art, can be utilized to obtain polymers with bound antioxidant at the same predetermined molecular weight, molecular weight distribution, stereo-specificity and co-monomer levels as for standard polymers. The molar ratio of catalyst components, that is base metal alkyl to transition metal component is unchanged. Also the procedure for addition of the catalyst components need not be altered. No pre-reaction of the base metal alkyl with the polymerizable antioxidant monomer is required, nor is one desired.

A useful example of Ziegler polymerization of the antioxidant of the present invention with a mixture of alpha-olefins is in the preparation of EPM rubbers and in the terpolymerization of ethylene, an alpha-olefin containing 3-18 carbon atoms, and a non-conjugated diene as in the preparation of EPDM rubbers. Suitable dienes in the preparation of such EPDM polymers include 1,4-hexadiene, monocyclic polyenes and polycyclic polyenes. Representative of such compounds include dicyclopentadiene, octadienes, cyclo-(2,2,1)-hepta-2,5-diene, the alkylidene norbornenes wherein the alkylidene group contains 1 to 20 carbon atoms and preferably 1 to 8 carbon atoms, the alkenyl norbornenes, and particularly the 5-alkenyl-2-norbornenes wherein the alkenyl group contains about 3 to 20 carbon atoms. Other suitable bridged ring polyenes include polyunsaturated derivatives of bicyclo-(2,2,2)-hexane such as bicyclo-(3,2,1)-hexane, polyunsaturated derivatives of bicyclo-(3,3,1)-nonane and polyunsaturated derivatives of bicyclo- (3,2,2)-nonane.

Examples of preferred bridged ring compounds include 5-methylene-2-norbornene, 5-ethylidene-2-norbornene, 5-vinyl-2-norbornene, 5-n-propylidene-2-norbornene, 5-isobutylidene-2-norbornene, 5-n-butylidene-2-norbornene, dicyclopentadiene, 5-(2-methyl-2-butenyl)-2-norbornene, 5-(3-methyl-2-butenyl)-2-norbornene and 5-(3,5-dimethyl-4-hexenyl)-2-norbornene.

The EPM/EPDM rubbers incorporating the polymerizable antioxidants of the present invention in the polymer backbone contain molar ratios of ethylene to propylene (or other of the C₃ to C₁₆ mono-olefins) varying between 95:1 to 5:90 of ethylene: propylene, and preferably between 85:15 to 55:45 of ethylene: propylene. The polyene or substituted polyene is chemically bound in the EPDM rubber in an amount within the range of 0 to 30 weight percent. The polymerizable antioxidant monomer of the present invention is bound into the polyolefin backbone in an amount ranging from 0.001 to 20 weight percent, depending in part on the particular use to which the polymer is put. The same amount of polymerizable antioxidant can be used in the other polymers as outlined above.

Such polymers are produced in an inter-polymerization in the presence of a Ziegler-catalyst well known to those skilled in the art. The techniques for producing such EPM or EPDM interpolymers is well known and is described in U.S. Patent Nos. 2,933,480, 3,093,621, 3,211,709, 3,646,168, 3,790,519, 3,884,993, 3,894,999 and 4,059,654, as well as many others.

The polymerizable antioxidant monomers described herein are also usefully employed in copolymerization with ethylene to produce high density polyethylene, including ultra high molecular weight polyethylene and with ethylene and alpha-olefins such as butene, hexene and octene to produce linear low density polyethylene. The reaction is carried out in accordance with standard conditions known to those skilled in the art with polymerizable antioxidant monomer being added as a solution of hexane, toluene, liquid monomer or aliphatic hydrocarbons.

Copolymerization of the polymerizable antioxidant monomers of the present invention with propylene or with propylene/alpha-olefin mixtures at a level of 0.01-1.0 weight percent produces thermoplastic propylene polymers resistant to auto-oxidation, and thereby capable of being processed under less restrictive conditions compared to conventional polypropylene type thermoplastics.

The polymerizable antioxidant of the present invention is thus copolymerized with the alpha olefin or mixtures of alpha olefins and serves to impart antioxidant properties to the polymer. One of the advantages of the present invention is that unlike antioxidants which are physically blended with a polymeric composition and have a tendency to migrate through the polymer matrix, the polymerizable antioxidants of the present invention do not undergo such migration because they are chemically bonded to the polymer matrix. The tendency to extract or leach out the antioxidant from fabricated articles where there is fluid contact during end use would also be overcome by practice of this invention. Incorporation of 0.1 to 2.0 weight percent polymerizable antioxidant monomer into the polyolefin backbone results in oxidative stability during isolation, transport and storage. Incorporation of levels of 0.5 to 20 weight percent polymerizable antioxidant monomer into the polyolefin backbone will provide oxidative stability for high temperature processing of the polyolefin. At these high levels of copolymerized antioxidant, the polyolefin will also be useful for blending with polyolefins and/or polymers not possessing antioxidant properties.

## Claims

1. A polyolefin prepared by Ziegler polymerization of an olefin selected from the group consisting of an alpha-olefin, a mixture of alpha-olefins, a diene, and a mixture of alpha-olefins and a diene or polyene with a polymerizable antioxidant monomer having the formula: 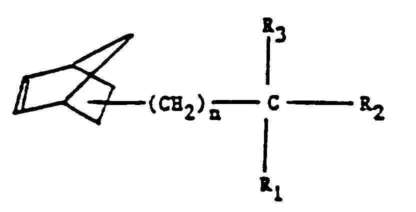 wherein n = 0-3; R₁ is an antioxidant-imparting substituent having the formula: 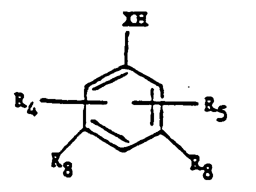 wherein X is selected from a divalent oxygen atom and a divalent sulfur atom; and R₄ and R₅ are each independently alkyl containing 1-8 carbon atoms or additionally R₄ can be hydrogen, R₈ is either hydrogen, methyl, ethyl or mixtures thereof, and R₂ is a hydrogen atom, and R₃ is either a hydrogen atom or an alkyl group.

2. A polyolefin according to claim 1, characterized in that R₄ and R₅ are selected from the group methyl, isopropyl or t-butyl.

3. A polyolefin according to anyone of claims 1-2, characterized in that X is oxygen.

4. A compound according to anyone of claims 1-3, characterized in that R₁ is a group having the formula

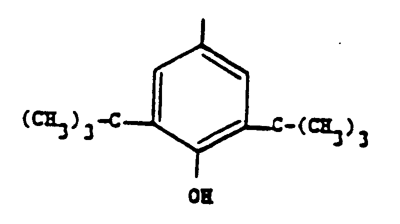

5. A polyolefin according to any one of claims 1-4, characterized in that the olefin is selected from the group ethylene, propylene, butene, 4-methyl-1-pentene, C₃-C₁₀ alpha olefins and mixtures thereof.

6. A polyolefin according to any one of claims 1-5, characterized in that the olefin is selected from the group of isoprene or butadiene.

7. A polyolefin according to claim 1, characterized in that the olefin is ethylene, propylene and at least one polyene.

8. A polyolefin according to claim 1, characterized in that the olefin is propylene and one or more dienes.

9. A polyolefin according to anyone of claims 1-8, characterized in that the antioxidant monomer is bound to the polyolefin in an amount ranging from 0.001-20 percent by weight.

10. A process for the polymerization to a polyolefin according to anyone of claims 1-9, characterized in that it comprises contacting
a) an olefin selected from the group consisting of an alpha-olefin, a mixture of alpha-olefins, a diene, and a mixture of alpha-olefins and a diene or polyene,
b) a compound having the formula: 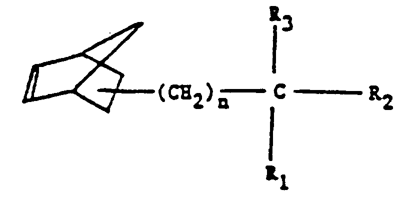 wherein n = 0-3; R₁ is an antioxidant-imparting substituent having the formula: 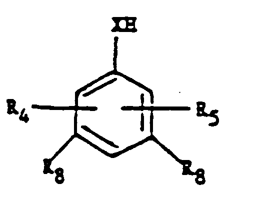 and R₂ is a hydrogen atom, wherein X is selected from a divalent oxygen atom and a divalent sulfur atom; and R₄ and R₅ are each independently alkyl containing 1-8 carbon atoms or additionally R₄ can be hydrogen, R₈ is either hydrogen, methyl, ethyl or mixtures thereof, and R₃ is either a hydrogen atom or an alkyl group,
c) in the presence of a Ziegler catalyst.
